# EUROPEAN PATENT SPECIFICATION

(11) **EP 2 968 972 B1**
(45) Date of publication and mention of the grant of the patent: **03.06.2020**
(21) Application number: 14759276.0
(22) Date of filing: 14.03.2014
(51) Int. Cl.: A61N 5/06, B82Y 20/00

(54) **QUANTUM DOT LIGHT-EMITTING DIODES FOR PHOTOTHERAPY**
QUANTENPUNKT-LEUCHTDIODEN FÜR EINE LICHTTHERAPIE
DIODES ÉLECTROLUMINESCENTES À POINT QUANTIQUE POUR PHOTOTHÉRAPIE

(30) Priority: 15.03.2013 US 201361799105 P
(43) Date of publication of application: 20.01.2016
(73) Proprietor: Nanoco Technologies, Ltd., Manchester M13 9NT (GB)
(72) Inventor: HARRIS, James, Manchester M14 6PE (GB); GLARVEY, Paul, Anthony, Stockport SK7 6JT (GB); MASALA, Ombretta, Manchester M14 6PE (GB); PICKETT, Nigel, Didsbury MA M20 6TR (GB); GRESTY, Nathalie, Chester CH3 5HG (GB)
(74) Representative: Dauncey, Mark Peter
(86) International application number: PCT/IB2014/001591
(87) International publication number: WO 2014/177943

(56) References cited:
- WO-A1-2012/110178
- WO-A2-2012/027728

## Description

### Background

Phototherapy, also known as heliotherapy, is the use of light to treat medical disorders. Modern-day phototherapy was pioneered by Niels Finsen, who was awarded the 1903 Nobel Prize for Physiology and Medicine for his work on the treatment of disease with concentrated light radiation. Finsen's initial work involved the separation of ultraviolet (UV) rays using quartz crystals, which was successful in the treatment of lupus vulgaris. Finsen also found that red light could be used to reduce scar formation in smallpox.

Since then, phototherapy has been used to treat a wide range of conditions, including skin disorders, circadian rhythm and seasonal affective disorders, neonatal jaundice, and tumours.

Treatment of skin conditions using phototherapy, e.g. psoriasis, eczema, dermatitis, acne vulgaris, is largely reliant on radiation in the UV region, however red to infrared (IR) light can be used to promote wound healing.

Hyperbilirubinaemia, better known as neonatal jaundice is a common condition, typically affecting over 50 % of newborn babies. Jaundice results from the inability of the liver to break down bilirubin, allowing it to accumulate in the blood. Phototherapy can be used to photo-oxidise *trans-*bilirubin to the water-soluble cis-isomer, which can then be more easily broken down by the liver and removed from the blood. According to the 2010 guidance set out by the National Institute of Health and Clinical Excellence (NICE) in the UK, treatment for neonatal jaundice depends on level of serum bilirubin. First-line treatment for babies born at or after 37 gestational weeks should be conventional "blue light" phototherapy, while for premature babies a fibre optic or "biliblanket" may be used in the first instance. Blue light is chosen to match the breakdown of bilirubin (which is optimal at 458 nm), though studies suggest that green light may be just as effective, thus providing a slightly less harmful alternative [H.Ayyash et al., Archives of Disease in Childhood, 1987, 62, 843]. It is suggested that since green light is more penetrating than blue light, this compensates for its poorer match to the absorption spectrum of bilirubin, [H.J. Vreman et al., Pediatric Research, 1998, 44, 804] which peaks at around 458 nm.

Phototherapy has shown some effect in the treatment of tumours. The action of light upon photosensitive molecules to induce toxicity was first observed by Oscar Raab around the turn of the 20^{th} Century [O. Raab, *Über die* Wirkung Fluoreszierender Stoffe auf Infusorien Z. Biol., 1900, 39, 524], when he noticed that a combination of light and acridine was toxic to the *paramecium* protozoa. In photodynamic therapy (PDT), which has been approved in the US by the Food and Drug Administration (FDA) for the treatment of lung cancer, the action of light upon a photoactive molecule is used to promote tumour necrosis *in vivo.* In addition to tumour treatment, NICE have implemented guidance for use of PDT in the UK in the treatment of pre-cancerous conditions such as Barrett's oesophagus, as well as benign conditions including wet age-related macular degeneration.

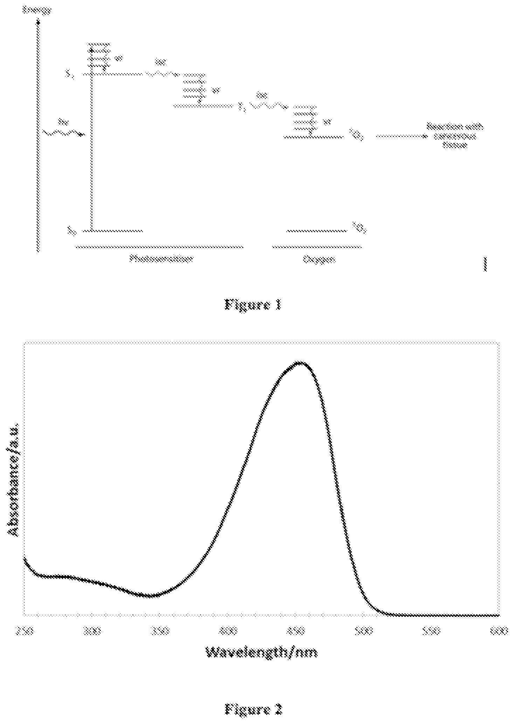

Since cancerous tissue is more responsive to the uptake of photosensitisers than healthy tissue, the approach can be used to selectively target cancerous cells. Selective photosensitiser uptake relies on the differences in chemical composition between healthy and tumorous tissues, which include the number of low-density lipoprotein receptors, the tissue pH, and water content. Suitable photoactive drugs can include porphyrin-based molecules, absorbing red light below 640 nm for treatment or tumours just beneath the surface of the skin or organ lining. For deeper penetration, IR-photoabsorbers are required. Chromophores within tissue, *i.e.* haemoglobin, melanin and water, will also absorb any incoming light. Thus, there is an "optical window" between 600-1300 nm, above the absorption spectrum of haemoglobin and below that of water, that is ideal for PDT. Photon absorption above 850 nm is often insufficiently energetic to produce singlet oxygen (see **Figure 1** for the mechanism of action), therefore photosensitisers that absorb strongly in the 600-850 nm range are most suitable for phototherapy. As such, there is a need for light sources emitting in this region.

Following *intravenous* (IV) administration of a photoactive substance, light is delivered using an endoscope or fibre-optic catheter. The mechanism of singlet oxygen generation is outlined in **Figure 1****;** upon photon absorption, the photosensitiser is excited from the ground state to the excited singlet state. After intersystem crossing to the excited triplet state, the energy is transferred from the photosensitiser to the excited singlet state of oxygen in the body. The singlet oxygen reacts with the cancerous biomolecules, resulting in cell death.

One first generation photosensitiser used for tumour phototherapy is haematoporphyrin derivative (HpD), which has five absorption bands at 400-410 nm, 500-505 nm, 535-540 nm, 565-575 nm and 620-635 nm. Since penetration depth decreases with decreasing wavelength, photoactivation is typically implemented with a 630 nm-emitting dye laser. Studies suggest that green light may be more efficient for treatment of tumours just below the surface of the skin or tissue [J.C. van Gemert et al., Br. J. Cancer, 1985, 52, 43]. Second and third generation photosensitisers absorb more strongly in the 650-850 nm range.

Light sources for PDT include lasers, discharge and fluorescent lamps, and light-emitting diodes (LEDs). The choice of light source depends on the depth of the legion, the absorption spectrum of the photosensitiser, and the attributes of the light source itself (reliability, ease of maintenance, cost, and size). At illumination rates in excess of 150 mW cm⁻², hyperthermia usually results, which can lead to oxygen depletion. Therefore, lower rates are often preferable. Hyperthermia should be especially avoided in the skin, since it causes increased pain. Thus high power laser outputs are not always favourable. In addition, broad spectrum light sources, such as halogen and metal halide lamps, may also be disadvantageous due to thermal effects and difficulty in controlling the light dosage. Consequently, there is a need for a narrow spectrum light source that emits in the 600-850 nm region, for which the power output can be readily controlled.

White light phototherapy is commonly used in the treatment of circadian rhythm disorders and seasonal affective disorder (SAD). It is believed that white light therapy early in the morning, during the winter months where there are fewer hours of daylight, can help to regulate the circadian rhythm and treat seasonal depressive symptoms. Currently, fluorescent light boxes are most commonly used, incorporating filters to block out harmful UV rays.

The therapeutic effects of light on the skin have more recently been extended to cosmetic applications. Perhaps the most well-known example is the sunbed. As the use of UV rays to stimulate melanin production in the skin falls out of favour owing to the increased risk of skin cancer, other cosmetic light therapies are being introduced to rejuvenate the skin.

In particular, red and IR light have been used for cosmetic phototherapy to improve the appearance of fine lines, wrinkles and skin tone. As with wound healing phototherapy, red to IR light is believed to stimulate collagen production and remodelling, resulting in smoother skin with a more even skin tone.

With such diverse applications, phototherapy relies on a wide range of wavelengths to target specific conditions. Current lamps rely on different light sources depending on the emission wavelength(s) required, limiting their use to very specific applications. As such, there is a need for a readily available means of converting the emission wavelength of a primary light source to emit at any given wavelength across the entire visible spectrum and into the IR.

WO2012/110178 describes a cell or cell tissue treatment device comprising an organic light emitting electrochemical cell, a light emitting electrochemical cell including at least one quantum dot, and an organic light emitting diode comprising at least one quantum dot. WO2012/027728 describes a composition comprising a cosmetically acceptable carrier and a plurality of plasmonic nanoparticles in a topical composition.

### Summary

Disclosed herein are articles for use in phototherapy utilizing quantum dots (QDs). In particular, disclosed herein is a medical device for phototherapy, the device comprising: a quantum dot (QD) light emitting diode (LED) chip package comprising a blue solid-state LED backlight optically coupled to a heavy metal-free QD, and wherein the medical device is configured as a dressing or shaped article dimensioned to provide photodynamic therapy to a patient in need thereof. One embodiment is a medical dressing having an occlusive layer and translucent layer. Quantum dot light-emitting diode chips are configured within the occlusive layer to provide light of a specific wavelength for use in phototherapy. Another embodiment is a medical dressing having an occlusive layer and translucent layer, wherein quantum dot material is embedded or impregnated within one or both layers.

### Brief Description of the Drawings

**Fig. 1** shows an energy level diagram for photodynamic therapy. Upon photon absorption, the photosensitiser is excited from the ground state (S₀) to the singlet state (S₁). After intersystem crossing (isc) to the excited triplet state of the photosensitiser (T₁), the energy is transferred to excited singlet oxygen (¹O₂). The singlet oxygen reacts with the cancerous biomolecules, resulting in cell death.
**Figure 2** is the absorption spectrum of bilirubin, showing broad blue-green absorption.
**Figure 3** is a diagram of a multi-layer dressing comprising an array of QD LED chips (and circuitry) embedded in an occlusive dressing, covered with a translucent dressing layer, for the administration of phototherapy to wounds.
**Figure 4** is a diagram of a white phototherapy lamp for the treatment of circadian rhythm disorders, comprising a primary light source and a transparent or translucent lampshade comprising QDs.
**Figure 5** shows the colour coordinates of a white light produced from a white LED and red and green QDs, in the Commission Internationale de l'Eclairage 1931 colour space. The correlated colour temperature (CCT) is 5,536 K and the white point lies close to the Planckian locus.
**Figure 6** shows the emission spectrum of an LED chip comprising a blue solid-state LED backlight and a red QD phosphor emitting at 625 nm. The relative peak intensities can be tuned by altering the QD concentration.
**Figure 7** shows the emission spectrum of an LED chip comprising a blue solid-state LED backlight and a red QD phosphor emitting at 653 nm. The relative peak intensities can be tuned by altering the QD concentration.

### Description

Disclosed herein are articles for use in phototherapy, which incorporate quantum dots (QDs). The wavelengths of light emitted by the QDs are tuned for specific phototherapies.

Examples of QDs and methods to prepare QD materials are provided in the Applicant's U.S. Patent Nos. 7,803,423, 7,985,466, 8,062,703, 7,588,828 and 7,867,556. The emission of QDs can be tuned across the entire visible spectrum and into the near-IR simply by changing the particle size. The high quantum yields and narrow band emission from QDs results in high colour purity with low energy loss from the solid-state backlight. Both the tunability and the colour quality and intensity of light available using QDs make QD materials promising materials for use in phototherapy.

Particular embodiments described herein use QDs to tune or "down convert" light from an LED light source to provide wavelengths of light needed for specific phototherapies. In the present invention, QDs are incorporated into an LED chip package with a blue solid-state LED backlight. The preparation of LED chip packages incorporating QDs is described in the Applicant's commonly owned U.S. Patent Application Pub. Nos. 2010/1023155 (published 20th May, 2010) and 2013/0140600 (published 6th June, 2013). Such LED chip packages may be incorporated into articles for use in specific modalities of phototherapy, as described below.

Alternatively, remote QD phosphors can be fabricated for use with a solid-state LED backlight. The preparation of remote QD phosphors is described in the applicant's co-pending GB Patent Application No. 1116517.2 (23rd September, 2011). In a remote phosphor configuration, the QD phosphor may take the form of an article that is in close proximity to, or in direct contact with, the human tissue such as the skin. Remote phosphor-based systems have the advantage that one remote phosphor can be substituted for another having a different colour of emission, thus a single LED backlight system can be used for treatment of a number of different conditions by simply swapping out remote phosphors.

One example of an article according to the present invention described herein is a medical dressing incorporating QDs. As used herein, the term "dressing" can refer to any article applied to the body to aid in phototherapy, but generally refers to a fabric or textile article, such as a bandage, blanket, article of clothing, sponge, compress, wrap, or the like. The dressing may incorporate LEDs, as described above, a remote phosphor, or may be impregnated, coated, or treated with a QD material. Each of these embodiments are discussed in more detail below, in the context of specific phototherapies

The articles herein described can encompass QDs emitting at one or more wavelengths depending on the application. The QDs can optionally be incorporated into beads prior to use, to enhance their stability. The devices utilise heavy metal-free QDs such as III-V (3-5) and I-III-VI₂ (1-3-6₂) semiconductor QDs, and including doped species and alloys thereof. The photoluminescence quantum yield (QY) of the nanoparticles can be improved by coating the QD "cores" with one or more shell layers of a wider bandgap material to form a core/(multi)shell QD. Beading and/or encapsulation techniques used in the device fabrication process significantly reduce the risk of exposure to toxic heavy metals from QDs, yet legislative restrictions on their use may make heavy metal-free QDs preferable, particularly for embodiments where the QDs are incorporated into an article that is in contact with human tissue.

### Treatment of hyperbilirubinaemia

The absorption spectrum of bilirubin is shown in Figure 2. Using green-emitting QD material with a blue solid-state LED backlight, emission can be tuned to the blue-green region of the EM spectrum for safe and effective treatment of neonatal jaundice. As used herein, the term "green QDs" refers to QDs that emit light in the green region of the visible spectrum when excited. Likewise, "red QDs" refers to QDs that emit red light, and so on. In certain embodiments, the QD material is incorporated into a secondary material or article for down-conversion of an external primary light source.

In one such example, green QDs are incorporated into a dye to stain a translucent or loose-weave textile material, which is used to produce bedding or a canopy that emits blue-green light when illuminated by a blue solid-state LED lamp. The QD dye is prepared by combining QDs with an appropriate solvent and, optionally, additives to modify, for example, the coating properties of the dye and/or its adherence to the textile. The QD dye is applied to the textile material by any suitable deposition method including, but not restricted to, immersing the material in the dye solution, drop casting, or inkjet printing. After applying the dye to the material, the material is allowed to dry, optionally with the application of gentle heat, to evaporate the solvent. Unlike with colour filters, for which unwanted wavelengths are absorbed, QD phosphors absorb high energy radiation and emit at a longer wavelength with relatively little loss of energy, determined by the photoluminescence QY of the nanoparticles. QYs greater than 80 % are well-documented in the prior art for core/(multi)shell QDs.

In an alternative embodiment, green QDs are incorporated into an incubator component, which is illuminated remotely using a blue solid-state LED lamp. The incubator component can be fabricated by incorporating QDs into the plastic from which it is constructed. For example, the Applicant's U.S. Patent No. 8,168,457 describes the preparation of a moulded QD-containing plastic material. Alternatively, the QDs can be applied as a coating to the outer and/or inner surface of a pre-fabricated incubator.

CdSe- and InP-based QDs, including doped species and alloys thereof, can be synthesised to provide blue to green emission. Examples of methods to prepare such QD materials are provided in the Applicant's U.S. Patent Nos. 7,803,423, 7,985,466, 8,062,703, 7,588,828 and 7,867,556. However, it will be appreciated that the embodiments described herein are not limited to QDs prepared by any specific method.

### Photodynamic Therapy

Using QDs it is possible to tune emission of a solid-state LED to match the peak wavelengths and relative intensities (by altering the relative concentrations of each colour of QDs) in the absorption spectrum of photosensitising molecules for the treatment of tumours and benign conditions such as wet age-related macular degeneration. Emission in the 600-850 nm "optical window" for PDT is possible using QDs. Using QDs it is also possible to achieve polychromatic emission from a single light source; by including the more activating but less penetrating wavelengths, *i.e.* blue-green emission, this may enhance tumour necrosis near the surface of the tissue, while the more penetrating wavelengths, *i.e.* red-IR, can still be emitted to target cells deeper in the tissue.

PDT systems may employ a fibre optic catheter to direct a specific wavelength of light towards a target tissue in the body. An optical fibre filled with QDs dispersed in a liquid support medium is taught in the European patent EP 0 783 784. By combining a solid-state light source, such as LEDs or laser light, with QDs emitting at a specific wavelength within the optical window for PDT, such a fibre optic device can be employed for PDT applications.

### Red and IR Phototherapy

Red and/or IR-emitting QDs may be incorporated into devices that can be applied to the skin for use in wound healing and cosmetic phototherapy. Using blue solid-state LEDs as the primary light source, red and/or IR QD phosphors may be used to down-convert the emission to stimulate collagen production and remodelling.

For the promotion of wound healing, QDs may be incorporated into a dye that is subsequently used to stain sterile gauze or loose-weave surgical dressings, allowing light from a primary light source (located near to or in the dressing, depending on the thickness) to excite the QDs in the dressing, which is in contact with the skin.

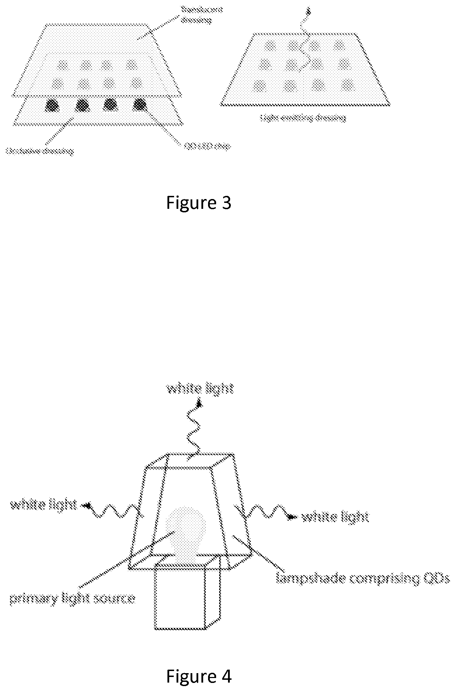

Alternatively, a multi-layer dressing may be assembled comprising an array of QD LED chips (and circuitry) embedded in an occlusive dressing layer, covered with a translucent dressing layer, as illustrated in **Figure 3****.** Light emitted by the QD LED chips passes through the translucent dressing layer to the wound. By embedding the QD LED chips into an occlusive dressing, the device enables the administration of phototherapy to wounds that must not be exposed to the air, for example to prevent infection.

In alternative embodiments, the QDs isincorporated into a transparent or translucent shaped article, as described in the Applicant's U.S. Patent No. 8,168,457. The shaped article may be moulded in a cast of the body part of the patient to whom it is to be applied. For example, for the rejuvenation of facial tissue, a shaped article in the form of a QD mask can be fabricated and applied to the face. The QD mask is then illuminated by a primary light source, such as a blue LED lamp. Upon irradiation, the QDs in the mask down-convert a proportion of the light to emit at wavelengths promoting skin rejuvenation.

### White Light Phototherapy

QDs can be used to fabricate LEDs emitting white light with a tunable correlated colour temperature (CCT), which can be used in the treatment of circadian rhythm and seasonal affective disorders. The CCT quantifies the "shade" of white light emitted, which can be tuned for therapeutic purposes. For example, a CCT close to that of natural daylight (round 5,500 - 6,500 K) can be beneficial for the treatment of circadian rhythm disorders such as seasonal affective disorder.

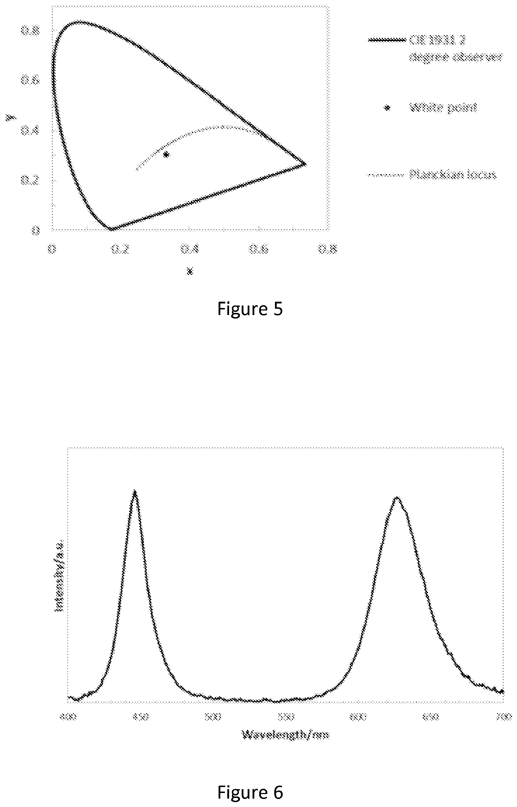

In some reference embodiments, a white-emitting phototherapy lamp is produced, comprising a primary light source and a lampshade comprising QDs, as shown in **Figure 4****.** The lampshade may be prepared by incorporating the QDs into a transparent or translucent shaped article, as described in the U.S. Patent No. 8,168,457. The QDs absorb a proportion of the primary light to produce white light with a desired colour temperature. In one reference embodiment, a phototherapy lamp comprises a blue LED primary light source in combination with a lampshade comprising red and green QDs to produce cool white light with a CCT in the range 5,500 - 6,500 K, to mimic natural daylight for the treatment of seasonal affective disorder. An example of a white lighting device comprising a blue LED backlight and red and green QDs, with a CCT of 5,536 K, is shown in **Figure 5**. The white point lies close to the Planckian locus (the path that a black body radiation source would take in chromaticity space as the colour temperature changes), indicating that the emitted light is close to that which would be emitted by the Sun at the same colour temperature. The CCT can be tuned by varying the emission wavelength of the QDs and/or the ratio of blue light:green QDs:red QDs. Suitable examples of red- and green-emitting nanoparticles include, but are not restricted to, CdSe and InP, and including doped species and alloys thereof.

### Examples

The tuning of the emission spectrum of QD LEDs for specific phototherapeutic applications is demonstrated in the following examples.

### Reference Example 1: QD LED Emitting within the Photodynamic Therapy Window

A QD LED chip comprising a blue solid-state LED as the primary light source and a red quantum dot silicone resin, with red emission in the optical window for PDT, was fabricated using CdSe/CdS/CdZnS/ZnS core-multishell QDs illuminated by a blue solid-state LED backlight emitting at 446 nm. The QD photoluminescence maximum (PLₘₐₓ) of 625 nm is within the red absorption band of HpD (620-635 nm), with a narrow FWHM of 35 nm. Therefore, it could be suitable for PDT with a porphyrin-derived photosensitiser as an alternative to a diode laser lamp. The relative blue and red peak intensities can be adjusted by altering the QD concentration.

The emission spectrum is shown in **Figure 6****.**

### Example 2 QD LED with Deep Red Emission to Promote Wound Healing

A QD LED chip comprising a blue solid-state LED as the primary light source and a red quantum dot silicone resin was fabricated according to the following procedure:

Silicone resin was mixed with a small amount of a Pt catalyst, then red InP/ZnS QD beads (20 ODs per 10 mmol solution in toluene) were added and the mixture was transferred to an LED case. The LED was cured under a nitrogen atmosphere.

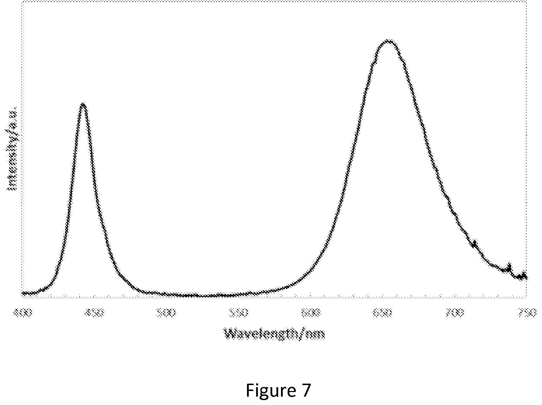

The QD LED was illuminated by a 22 mW blue solid-state LED. Blue emission was seen around 442 nm from the solid-state LED. The red QD material emitted with PLmax = 653 nm and FWHM = 60 nm. This deep red emission could be suitable for wound healing or cosmetic phototherapy. The relative intensity of the blue (LED) to red (QD phosphor) light was 1:1.34, but could be adjusted by altering the QD concentration. The emission spectrum is shown in **Figure 7****.**

The invention has been described herein with reference to specific non-limiting embodiments. It will be appreciated that many modifications and permutations are possible without deviating from the scope of the invention.

## Claims

1. A medical device for phototherapy, the device comprising:
a quantum dot (QD) light-emitting diode (LED) chip package comprising a blue solid-state LED backlight optically coupled to a heavy metal-free QD, and wherein the medical device is configured as a dressing or shaped article dimensioned to provide photodynamic therapy to a patient in need thereof.

2. A medical device as recited in Claim 1, wherein the quantum dot comprises CuInS₂, CuInSe₂, CuGaS₂, CuGaSe₂, or InP/ZnS quantum dot materials, or their doped or alloyed quantum dot derivatives.

3. The medical device recited in claim 1, wherein the QD LED chip packages comprise red- or infrared-emitting quantum dots.

4. The medical device recited in claim 1, wherein the quantum dots comprise a core and one or more shell layers formed on the core.

5. The medical device recited in claim 1, wherein the quantum dots are selected from quantum dots that emit light in a wavelength range from 600 to 850 nm.

6. The medical device recited in claim 1, wherein the quantum dots comprise a semiconductor material formed from Group 3 - 5 elements of the periodic table of the elements, or their doped or alloyed derivatives.

7. The medical device recited in claim 1, wherein the device comprises an occlusive layer and a translucent layer and the quantum dots are configured within the occlusive layer.

8. The medical device recited in claim 1, wherein QD LED chip packages comprise green or blue-green emitting quantum dots.

## Patentansprüche

1. Medizinische Vorrichtung für eine Lichttherapie, wobei die Vorrichtung Folgendes beinhaltet:
eine Quantenpunkt (QD)-Leuchtdioden (LEd)- Chip-Packung, beinhaltend eine blaue Feststoff-LED-Hintergrundbeleuchtung, welche optisch mit einem schwermetallfreien QD gekoppelt ist, und wobei die medizinische Vorrichtung als Verband oder geformter Artikel konfiguriert ist, welcher dimensioniert ist, um eine photodynamische Therapie an einen Patienten bereitzustellen, der diese benötigt.

2. Medizinische Vorrichtung nach Anspruch 1, bei welcher der Quantenpunkt CuInS₂-, CuInSe₂-, CuGaS₂-, CuGaSe₂- oder InP/ZnS-Quantenpunkt-Materialien, oder deren dotierte oder legierte Derivate beinhaltet.

3. Medizinische Vorrichtung nach Anspruch 1, bei welcher die QD-LED-Chip-Packungen Rot oder Infrarot emittierende Quantenpunkte beinhalten.

4. Medizinische Vorrichtung nach Anspruch 1, bei welcher die Quantenpunkte einen Kern und eine oder mehrere am Kern gebildete Schalenlage(n) beinhalten.

5. Medizinische Vorrichtung nach Anspruch 1, bei welcher die Quantenpunkte aus Quantenpunkten gewählt sind, welche Licht in einem Wellenlängenbereich von 600 bis 850 nm emittieren.

6. Medizinische Vorrichtung nach Anspruch 1, bei welcher die Quantenpunkte ein Halbleitermaterial beinhalten, welches aus Elementen der Gruppen 3-5 des Periodensystems der Elemente oder aus deren dotierten oder legierten Derivaten gebildet ist.

7. Medizinische Vorrichtung nach Anspruch 1, bei welcher die Vorrichtung eine Verschlussschicht und eine durchscheinende Schicht beinhaltet und die Quantenpunkte innerhalb der Verschlussschicht konfiguriert sind.

8. Medizinische Vorrichtung nach Anspruch 1, bei welcher die QD-LED-Chip-Packungen Grün oder Blaugrün emittierende Quantenpunkte beinhalten.

## Revendications

1. Dispositif médical pour photothérapie, le dispositif comprenant :
un ensemble de puce à diode électroluminescente (LED) à point quantique (QD) comprenant un rétroéclairage à LED à semi-conducteurs bleu raccordé d'un point de vue optique à un QD sans métal lourd, et dans lequel le dispositif médical est configuré comme un pansement ou un article façonné dimensionné de façon à fournir un traitement photodynamique à un patient en ayant besoin.

2. Dispositif médical selon la revendication 1, dans lequel le point quantique comprend des matériaux de point quantique de CuInS₂, du CuInSe₂, du CuGaS₂, du CuGaSe₂, ou d'InP/SnS, ou leurs dérivés de point quantique dopés ou alliés.

3. Dispositif médical selon la revendication 1, dans lequel les ensembles de puce de LED QD comprennent des points quantiques émettant de la lumière rouge ou infrarouge.

4. Dispositif médical selon la revendication 1, dans lequel les points quantiques comprennent un noyau et une ou plusieurs couche(s) de coque formée(s) sur le noyau.

5. Dispositif médical selon la revendication 1, dans lequel les points quantiques sont sélectionnés à partir de points quantiques qui émettent de la lumière dans une plage de longueur d'ondes de 600 à 850 nm.

6. Dispositif médical selon la revendication 1, dans lequel les points quantiques comprennent un matériau à semi-conducteur formé à partir des éléments des Groupes 3 - 5 de la table périodique des éléments, ou leurs dérivés dopés ou alliés.

7. Dispositif médical selon la revendication 1, dans lequel le dispositif comprend une couche occlusive et une couche translucide et les points quantiques sont configurés dans la couche occlusive.

8. Dispositif médical selon la revendication 1, dans lequel les ensembles de puce à LED QD comprennent des points quantiques émettant une lumière verte ou bleue-verte.
